# EUROPÄISCHE PATENTSCHRIFT

(11) **EP 0 839 063 B1**
(45) Veröffentlichungstag und Bekanntmachung des Hinweises auf die Patenterteilung: **29.12.1999**
(21) Anmeldenummer: 96923824.5
(22) Anmeldetag: 05.07.1996
(51) Int. Cl.: A61M 39/06

(54) **DICHTVENTIL, INSBESONDERE FÜR DIE KATHETERTECHNIK**
SEALING VALVE, IN PARTICULAR USED IN THE CATHETER TECHNIQUE
SOUPAPE D'ETANCHEITE, NOTAMMENT POUR CATHETERISME

(30) Priorität: 18.07.1995 DE 19526075
(43) Veröffentlichungstag der Anmeldung: 06.05.1998
(73) Patentinhaber: Wollschläger, Helmut, Dr., 79102 Freiburg (DE)
(72) Erfinder: Wollschläger, Helmut, Dr., 79102 Freiburg (DE)
(74) Vertreter: Rackette, Karl, Dipl.-Phys. Dr.-Ing
(86) Internationale Anmeldenummer: DE9601245
(87) Internationale Veröffentlichungsnummer: WO9703719

(56) Entgegenhaltungen:
- EP-A- 0 267 584
- EP-A- 0 534 000
- EP-A- 0 546 712
- WO-A-94/14497

## Beschreibung

Die Erfindung betrifft ein Dichtventil mit einem langgestreckten, ein Anschlußende und ein Dichtende aufweisenden Ventilkörper, in dem ein sich vom Anschlußende zum Dichtende in axiale Richtung erstreckende Ventildurchführung ausgebildet ist, in der ein mit einer axialen Pfropfendurchführung versehener, in axiale Richtung zum Dichten komprimierbarer Dichtpfropfen angeordnet ist.

Ein derartiges Dichtventil ist aus der EP 0 267 584 bekannt. Der Ventilkörper des bekannten Dichtventils weist ein das Anschlußende bildendes Hauptrohr auf, an das ein das Dichtende bildender Dichtzylinder angesetzt ist. Der Innendurchmesser des Dichtzylinders ist größer als der Innendurchmesser des Hauptrohres. Der Übergang vom Innenraum des Dichtzylinders zur Durchführung des Hauptrohres ist über einen konisch zulaufenden Schulterabschnitt bewerkstelligt. Im Innenraum des Dichtzylinders befindet sich ein am konischen Schulterabschnitt anliegender, elastischer Dichtpfropfen, der mit Hilfe eines in axiale Richtung beweglichen, jedoch gegen Drehungen im Innenraum des Dichtzylinders gesicherten Stempels komprimierbar ist. Der Stempel ist auf seiner zum Dichtende hin weisenden Seite mit einer Spiralfläche versehen, auf die eine weitere Spiralfläche eines auf das Dichtende des Dichtzylinders aufgesetzten Hebelverschlusses drückt. Um das Ventil zu betätigen, wird der Hebelverschluß gegenüber dem Dichtzylinder gedreht, so daß sich die Spiralflächen gegeneinander verschieben und der Stempel in axiale Richtung bewegt wird. Ist der Dichtpfropfen komprimiert, weicht das elastische Material des Dichtringes nach innen aus und die Pfropfendurchführung verkleinert sich. Dadurch ist der Dichtpfropfen in der Lage, das Dichtventil oder ein durch das Dichtventil hindurch geführtes Instrument abzudichten.

Ein derartiges Dichtventil ist ein wesentlicher Bestandteil der interventionellen Kathetertechnik. Diese Technik wird beispielsweise zum Aufweiten verengter Herzkranzadern mit Hilfe eines Ballonkatheters angewandt. Um den Ballonkatheter an den zu behandelnden Ort der Herzkranzader zu bringen, muß zunächst ein Führungskatheter durch eine Öffnung in der Leistengegend des Patienten über die Hauptschlagader bis zum Abgang der zu behandelnden Arterie vorgeführt werden. Danach wird ein Draht mit einem Durchmesser von wenigen Zehnteln Millimetern durch diesen Führungskatheter hindurch in die zu behandelnde Ader eingeführt. Auf diesen Draht wird daraufhin der Ballonkatheter aufgefädelt und zum Ort der Aufdehnung vorgeschoben.

Während dieser Behandlungsschritte ist der Führungskatheter durch das Dichtventil abgedichtet. Beim Einführen der Instrumente hält die linke Hand des behandelnden Arztes das Dichtventil, während die rechte Hand die Handgriffe zum Manövrieren der Instrumente durchführt und zwischendurch das Ventil entweder zu- oder aufschraubt. Wegen des zum Betätigen des Ventils notwendigen umständlichen Umgreifens der rechten Hand bleibt das Ventil öfters unbeabsichtigt und unnötig geöffnet, weswegen der Patient mitunter erheblich Blut verliert. Dieser Sachverhalt stellt unter Umständen eine Gefährdung für den Patienten dar. Auf jeden Fall wird das Instrumentierfeld stark mit Blut verschmutzt.

Weiter ist aus der US 4,917,668 ein Dichtventil bekannt, das von einem Dichtkörper gebildet ist. Der Dichtkörper weist ein an einen Führungskatheter anschließbares Anschlußteil auf, an das sich in axiale Richtung ein Einführteil anschließt. Das Anschlußteil und das Einführteil sind jeweils mit einer zentralen Durchführung versehen, die durch eine im Bereich der Durchführung mit Schlitzen versehene und zwischen Anschlußteil und Einführteil eingeklemmte, elastische Dichtmembran abdichtbar ist. Auf der zum anzuschließenden Katheter weisenden Seite der Dichtmembran ist die Dichtmembran durch ein im Anschlußteil angeordnetes Federelement abgestützt, das den Verschluß der Dichtmembran gewährleistet, wenn sich kein abzudichtender Gegenstand in der Durchführung befindet. Auf der dem anzuschließenden Katheter abgewandten Seite der Dichtmembran befindet sich ein in seiner Ruhestellung durch ein weiteres Federelement im Abstand zur Dichtmembran gehaltener Stößel mit einer zentralen Durchführung, der zum Öffnen der Dichtmembran in axiale Richtung auf die Dichtmembran zu bewegbar ist. Zum Öffnen des Dichtventils wird in das Einführteil ein Führungsrohr eingeschoben, das den Stößel durch die Dichtmembran hindurchschiebt. Durch die auf diese Weise geöffnete Dichtmembran und die Durchführungen des Dichtkörpers ist daraufhin ein Katheter in den am Anschlußteil des Dichtkörpers angebrachten Führungskatheter einschiebbar.

Aus der EP 0 135 140 ist ein Rückschlagventil für medizinische Zwecke bekannt, daß ein topfartiges Ventilgehäuse aufweist. Am Topfboden ist eine Spiralfeder angebracht, die einen Ventilschieber gegen einen im Bereich der Topföffnung angeordneten Dichtanschlag drückt. Der in axiale Richtung im Ventilgehäuse verschiebbare Schieber übersteuert mit seiner Umfangsfläche eine in der Wand des Ventilgehäuses eingebrachte Ventilöffnung, die zu einer in axialer Richtung verlaufenden, in die Außenseite des Ventilgehäuses eingebrachte Kanalnut führt. Zum Öffnen des Ventils wird der Schieber in Richtung des Topfbodens verschoben, bis der Schieber die Ventilöffnung in der Wand des Ventilgehäuses freigibt und das abzudichtende Fluid von der Topföffnung des Ventilgehäuses durch die Ventilöffnung und die Kanalnut in einen das Ventilgehäuse umschließenden Katheter strömen kann.

Ausgehend von diesem Stand der Technik liegt der Erfindung die Aufgabe zugrunde, ein Dichtventil zu schaffen, das feinfühlig und zeitnah auf ergonomische Weise mit einer Hand betätigbar ist.

Diese Aufgabe wird dadurch gelöst, daß zum Komprimieren des Dichtpfropfens ein durch einen Betätigungsmechanismus in seiner Wirkung neutralisierbares Federelement vorgesehen ist.

Dadurch daß die Kraft zum Komprimieren des Dichtpfropfens von einem Federelement aufgebracht wird, läßt sich das Dichtventil rasch öffnen und schließen. Außerdem ist gewährleistet, daß sich das Dichtventil beim Loslassen des Betätigungsmechanismus selbsttätig schließt.

Bei einer bevorzugten Ausgestaltung drückt ein längs zum Ventilkörper verlaufender und am Ventilkörper verschwenkbar gelagerter Hebelarm mit einer rechtwinklig am Hebelarm angesetzten Druckgabel auf einen in die Durchführung des Ventilkörpers eingreifenden, am Dichtpfropfen anliegenden Druckkolben. Die Federkraft wird durch ein zwischen Hebelarm und Ventilkörper eingespanntes Federelement aufgebracht.

Dieses Dichtventil läßt sich auf entspannte Weise mit einer Hand, meistens der linken Hand, halten. Dabei liegen Zeigefinger, Mittelfinger und Ringfinger auf dem Hebelarm und drücken das Dichtventil gegen den auf der gegenüberliegenden Seite des Ventilkörpers anliegenden Daumen. Durch Zusammendrücken von Zeige-, Mittel- und Ringfinger einerseits und Daumen andererseits ist das Ventil auf ergonomische Weise durch eine einzige Bewegung mit einer Hand betätigbar.

Da es sich bei diesem Dichtventil um einen in großen Stückzahlen gefertigten Wegwerfartikel handelt, ist der zur Lagerung benötigte Raum ein wesentlicher Gesichtspunkt. Der Hebelarm ist bei diesem Dichtventil längs zum Ventilkörper angeordnet. Dadurch ist das benötigte Lagervolumen auch bei großer Hebelarmlänge klein.

Mit einer großen Hebellänge aber läßt sich die nötige Feinfühligkeit beim Öffnen oder Schließen erreichen.

Nachfolgend werden vorteilhafte Ausgestaltungen der Erfindung anhand der Zeichnung beschrieben. Es zeigen:
- Fig. 1: eine Längsschnittansicht eines Dichtventils im geschlossenen Zustand;
- Fig. 2: eine Längsschnittansicht des Dichtventils im geöffneten Zustand;
- Fig. 3: eine Ansicht von hinten des Dichtventils aus den Fig. 1 und 2 in geschlossenen Zustand;
- Fig. 4: eine Längsschnittansicht einer weiteren Ausgestaltung des Dichtventils im geschlossenen Zustand;
- Fig. 5: eine Längsschnittansicht des Dichtventils aus Fig. 4 im geöffneten Zustand;
- Fig. 6: einen Querschnitt durch den Hebelarm entlang der Schnittlinie VI-VI in Fig. 4;
- Fig. 7: eine weitere Ausgestaltung des Dichtventils;
- Fig. 8: einen Querschnitt durch den Hebelarm entlang der Schnittlinie VIII-VIII in Fig. 7;
- Fig. 9: eine Längsschnittansicht durch das Dichtventil aus Fig. 7 im geöffneten Zustand;
- Fig. 10: einen Querschnitt durch den Hebelarm entlang der Schnittlinie X-X in Fig. 9;
- Fig. 11: eine Ansicht von hinten auf das Dichtventil aus Fig. 9; und
- Fig. 12: ein weiteres abgewandeltes Ausführungsbeispiel des Dichtventils.

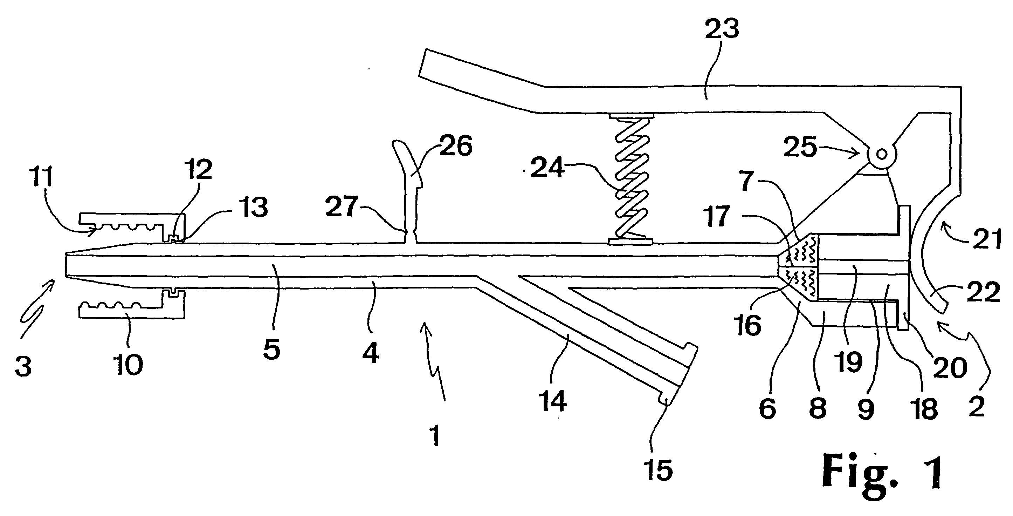

Fig. 1 zeigt eine Querschnittsansicht des Dichtventils, das einen Ventilkörper 1 mit einem Dichtende 2 und einem Anschlußende 3 aufweist. Der Ventilkörper 1 verfügt über ein Hauptrohr 4 mit einer axialen Durchführung 5. An das Hauptrohr 4 ist über eine konische Erweiterung 6 mit einer konischen Innenfläche 7 ein Dichtzylinder 8 mit einem Innenraum 9 angesetzt. Am Anschlußende 3 ist das Hauptrohr 4 konisch angeschrägt, um den Anschluß an einen Führungskatheter zu ermöglichen. Außerdem befindet sich am Anschlußende 3 eine Schraubmanschette 10, die über ein Innengewinde 11 auf ein Außengewinde des Führungskatheters aufschraubbar ist. Eine in der Zeichnung durch eine Ringnut 12 und eine Ringfeder 13 angedeutete Dichtung sorgt für eine dichte Verbindung des Dichtventils mit dem Führungskatheter. Im Abstand zum Anschlußende 3 ist seitlich an das Hauptrohr 4 im spitzen Winkel zum Hauptrohr 4 ein Seitenrohr 14 angesetzt, das über einen Flansch 15 beispielsweise an ein Druckmeßgerät anschließbar ist. An der konischen Innenfläche 7 der konischen Erweiterung 6 liegt ein elastischer Dichtpfropfen 16 an, der die Gestalt eines Zylinders mit einem aufgesetzten Konus hat. In dem Dichtpfropfen 16 ist eine axiale Pfropfendurchführung 17 ausgebildet. In der in Fig. 1 dargestellten geschlossenen Stellung ist der Dichtpfropfen 16 durch einen Druckkolben 18 zusammengedrückt und die Pfropfendurchführung 17 ist geschlossen. Genauso wie der Dichtpfropfen 16 verfügt der Druckkolben 18 über eine axiale Kolbendurchführung 19. Nach außen hin ist am Druckkolben 18 eine einen Anschlag gegen den Dichtzylinder 8 bildende Druckplatte 20 ausgebildet. Auf die Druckplatte 20 drückt eine Druckgabel 21 mit gekrümmten Zinken 22. Die Druckgabel 21 ist rechtwinklig an einen seitlich in Längsrichtung zum Hauptrohr 4 angeordneten Hebelarm 23 angesetzt. Bewegt sich der Hebelarm 23, rollen die Zinken 22 der Druckgabel 21 auf der Druckplatte 20 ab. Zwischen dem Hebelarm 23 und dem Hauptrohr 4 ist eine Schraubendruckfeder 24 eingespannt. Der Deutlichkeit halber ist die Schraubendruckfeder 24 nicht im Querschnitt, sondern in einer Seitenansicht dargestellt. Der Hebelarm 23 ist über ein im Bereich des Dichtendes 2 angeordnetes Scharniergelenk 25 verschwenkbar gelagert. Die Schraubendruckfeder 24 drückt den Hebelarm 23 vom Hauptrohr 4 weg, und damit drücken die Zinken 22 der Druckgabel 21 auf die Druckplatte 20 des Druckkolbens 18, der den Dichtpfropfen 16 zusammendrückt. Das Material des elastischen Dichtpfropfens 16 weicht von der konischen Innenfläche 7 geführt nach innen aus und verschließt dadurch die Pfropfendurchführung 17. Ein durch die Pfropfendurchführung 17 hindurchgeführtes Instrument ist dadurch abgedichtet.

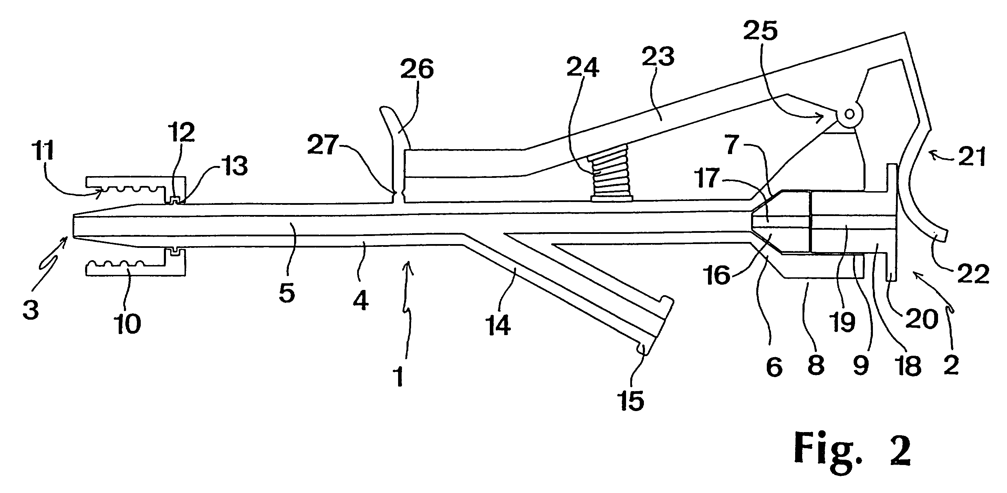

Fig. 2 zeigt das Dichtventil aus Fig. 1 im geöffneten Zustand. Die Schraubendruckfeder 24 ist zusammengedrückt und das Ende des Hebelarms 23 ist mit Hilfe eines Schnapphakens 26 arretiert. Der am Hauptrohr 4 angebrachte Schnapphaken 26 weist eine Biegeverjüngung 27 auf. Durch diese Biegeverjüngung 27 ist der Schnapphaken 26 zurückziehbar, und der Hebelarm 23 läßt sich aus der Arretierung lösen. Somit läßt sich durch Betätigen des Hebelarms 23 die Wirkung der Schraubendruckfeder 24 neutralisieren.

Es sei angemerkt, daß bei einem abgewandelten, in der Zeichnung nicht dargestellten Ausführungsbeispiel der Schnapphaken 26 zwei- oder mehrstufig ausgebildet ist. Eine derartige Ausbildung des Schnapphakens 26 erlaubt dem Benutzer, das Ventil stufenweise zu öffnen oder zu schließen.

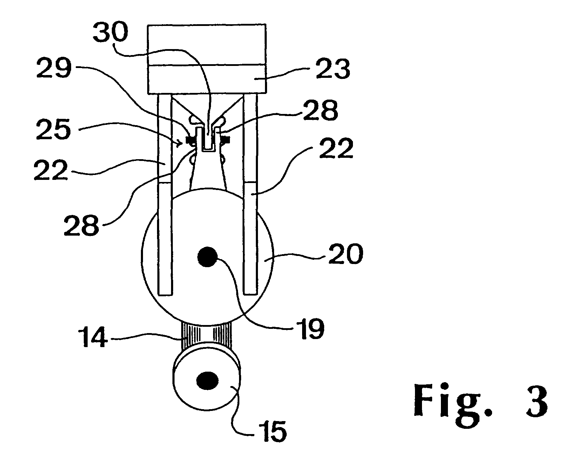

Fig. 3 zeigt eine Ansicht des Dichtendes 2 des Dichtventils. Man erkennt deutlich die Druckplatte 20 mit der zentralen Kolbendurchführung 19. Außerdem sind die beidseitig der Kolbendurchführung 19 auf der Druckplatte 20 anliegenden Zinken 22 der Druckgabel 21 erkennbar. Zwischen den Zinken 22 der Druckgabel 21 ist das Scharniergelenk 25 zu sehen. Zwei am Dichtzylinder 8 angebrachte Gelenkstreben 28 halten einen Gelenkstift 29. Zwischen die Gelenkstreben 28 greift eine an dem Hebelarm 23 angebrachte Gelenköse 30 ein, die auf dem Gelenkstift 29 verschwenkbar gelagert ist. Hinter dem Scharniergelenk 25 ist die Schraubendruckfeder 24 erkennbar.

Um den Hebelarm 23 seitlich abzustützen, sind bei einem abgewandelten, in der Zeichnung nicht dargestellten Ausführungsbeispiel die Gelenkstreben 28 und die Gelenköse 30 in Längsrichtung verlängert, so daß stegförmige Gelenkstreben eine Nut bilden, in die eine stegförmige Gelenköse eingreift.

Das in den Fig. 1 bis 3 dargestellte Dichtventil wird vor allem im Rahmen der interventionellen Kathetertechnik angewandt. Um beispielsweise Verengungen der Herzkranzadern mit Hilfe eines Ballonkatheters aufzuweiten, muß der Ballonkatheter an den Ort der Herzkranzader gebracht werden. Dazu wird zunächst ein stabiler Führungskatheter, mit einem verhältnismäßig großen Lumen von bis zu zehn French (= drei Millimeter) durch eine Öffnung in der Leistengegend des Patienten über die Hauptschlagader bis zum Abgang der zu behandelnden Herzkranzader vorgeführt. Der Führungskatheter wird durch das Dichtventil gemäß der Erfindung abgedichtet. Danach wird ein dünner Führungsdraht von einem French (= ein Drittel Millimeter) Durchmesser durch das Dichtventil und den Führungskatheter bis weit in die zu behandelnde Herzkranzader eingeführt. Über diesen Draht wird dann der Ballonkatheter, der der eigentlichen Behandlung dient, aufgefädelt und bis zum Ort der Aufdehnung vorgeschoben. Während der gesamten Behandlung ist der Führungskatheter gegenüber dem hohen Blutdruck in der Schlagader (150 mm Hg-Säule ≈ 0,2 Bar) durch ein Dichtventil gemäß der Erfindung abgedichtet.

Es sei angemerkt, daß in der Radiologie Führungskatheter mit einem Lumen von bis zu 14 French (= 4,2 Millimeter) Durchmesser zum Aufweiten von Adern verwendet werden. Auch derartige Führungskatheter lassen sich mit dem Dichtventil gemäß der Erfindung abdichten.

Während der Behandlung hält die linke Hand des behandelnden Arztes das Dichtventil, während die rechte Hand die Handgriffe zum Manövrieren der Instrumente durchführt. Das Dichtventil läßt sich auf besonders ergonomische Weise betätigen, wenn das Dichtventil zwischen Daumen einerseits und Zeige-, Mittel- und Ringfinger andererseits gehalten ist. Bei diesem Griff müssen zum Halten des Dichtventils lediglich Zeige-, Mittel- und Ringfinger leicht gekrümmt werden. Zum Schließen des Dichtventils werden Zeige-, Mittel- und Ringfinger gegen den Daumen in einer einzigen Bewegung zusammengedrückt. Da nur eine einzige Handbewegung zum Betätigen des Ventils nötig ist, ist ein entspanntes Arbeiten möglich.

Außerdem läßt sich das Dichtventil zeitnah betätigen. Beim Durchführen der Behandlung hat der behandelnde Arzt aus psychologischen Gründen öfters den Eindruck, daß das in die zu behandelnde Ader vorgeschobene Instrument durch die Dichtung des Dichtventils blockiert ist, wohingegen in Wirklichkeit die hemmende Reibung an einem Ort im Körper des Patienten erzeugt wird. Mit einem Dichtventil, das sich rasch öffnen und schließen läßt, kann der behandelnde Arzt rasch die Ursache der Reibung feststellen, indem er das Dichtventil für kurze Zeit leicht öffnet. Falls dadurch der die Bewegung des Instruments hemmende Widerstand nicht abnimmt, liegt die den Widerstand erzeugende Stelle im Körper des Patienten. Davon kann sich der Arzt umso leichter vergewissern, als das Dichtventil mit einer einzigen Hand betätigbar ist, so daß ein zeitraubendes Umgreifen vom zu manövrierenden Instrument zum Dichtventil unnötig ist.

Ein weiterer Vorteil ist, daß das Dichtventil im Ruhezustand geschlossen ist. Somit kann der behandelnde Arzt, wenn er eine Ruhepause benötigt, das Dichtventil einfach loslassen, ohne daß er weitere Handgriffe zum Schließen des Dichtventils ausführen muß.

Das Dichtventil ist vorteilhafterweise als Wegwerfgegenstand ausgeführt. Dementsprechend besteht die Mehrzahl der Bauelemente des Dichtventils aus Kunststoff. Falls es zur Kraftübertragung nötig ist, sind außer der Schraubdruckfeder 24 weitere Bauelemente, wie der Hebelarm 23 oder die Zinken 22 der Druckgabel 21, aus einem metallischen Werkstoff gefertigt.

Da das Dichtventil als Wegwerfartikel in großen Stückzahlen hergestellt wird, ist es von Vorteil, wenn für die Lagerung wenig Platz erforderlich ist. Bei dem Dichtventil gemäß der Erfindung ist der Hebelarm 23 längs zum Hauptrohr 4 angeordnet. Folglich vergrößert auch ein langer Hebelarm 23 das zur Lagerung erforderliche Volumen nicht wesentlich.

Ein langer Hebelarm ist jedoch nötig, im die notwendige Feinfühligkeit beim Betätigen des Ventils zu erreichen.

Eine Reihe von Abwandlungen des Dichtventils gemäß der Erfindung sind denkbar.

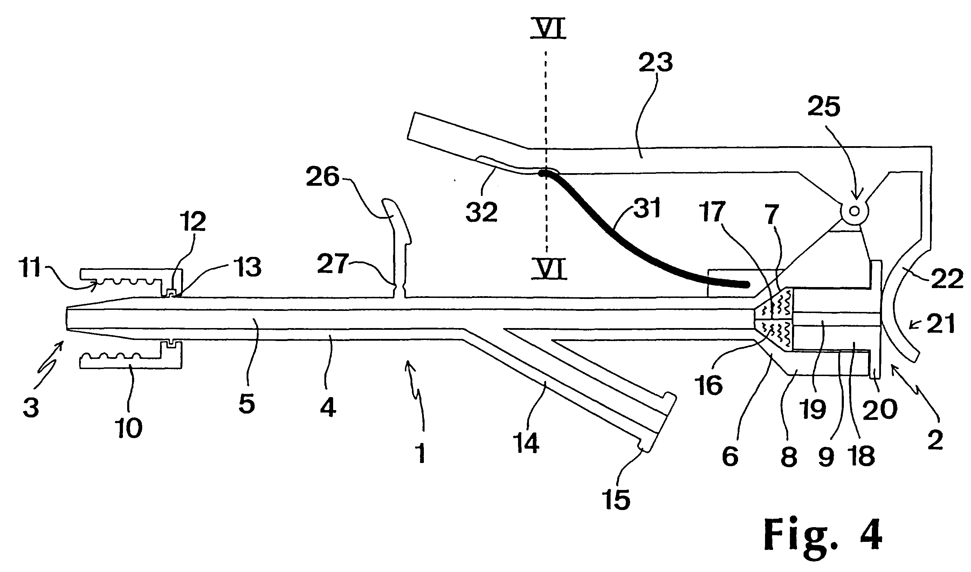

Fig. 4 zeigt ein Dichtventil, bei dem die Schraubendruckfeder 24 durch eine Blattfeder 31 ersetzt ist. Die Blattfeder 31 ist an einem Ende im Bereich des Dichtzylinders 8 am Ventilkörper 1 befestigt, während das andere Ende der Blattfeder 31 in einer im Hebelarm 23 ausgebildeten Führungsnut 32 gleitet.

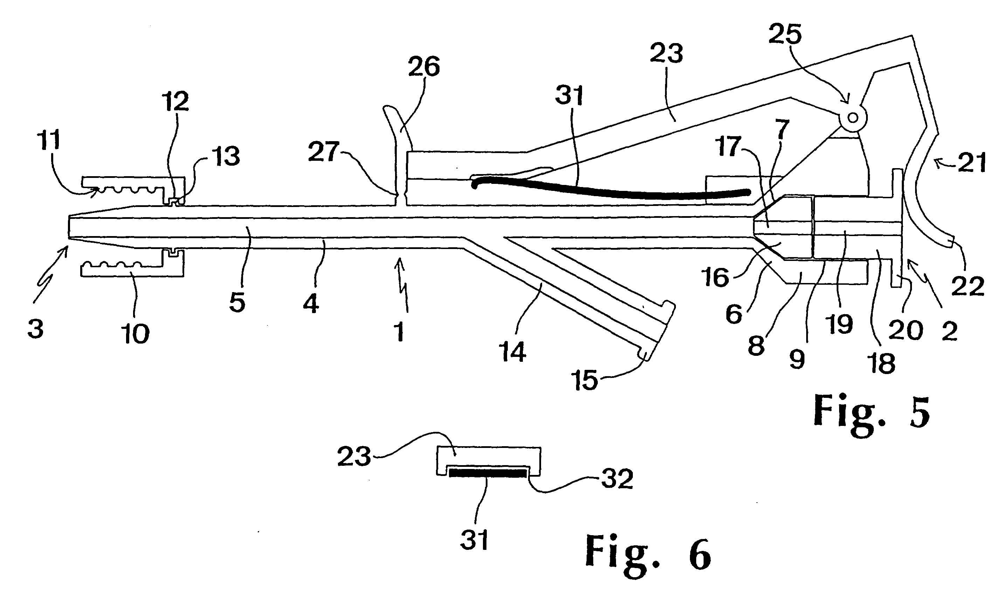

Fig. 5 zeigt das Ausführungsbeispiel aus Fig. 4 im geöffneten Zustand. Man erkennt, daß der Hebelarm 23 wie bei dem anhand der Fig. 1 bis 3 beschriebenen Ausführungsbeispiel durch einen Schnapphaken 26 arretierbar ist.

Fig. 6 zeigt zur Verdeutlichung einen Querschnitt durch den Hebelarm 23 entlang der Linie VI-VI in Fig. 4. Man erkennt die Blattfeder 31, die in der Führungsnut 32 gleitet. Die Führungsnut 32 dient vor allem dazu, seitliche Bewegungen des Hebelarmes 23 zu verhindern. Falls sich durch diese Maßnahme nicht eine ausreichende Seitenstabilität erzielen läßt, läßt sich die Führung des Hebelarmes 23 dadurch verbessern, daß, wie oben erwähnt, die Gelenkstreben 28 sowie die Gelenköse 30 in Längsrichtung des Ventilkörpers 1 verlängert sind, so daß der Hebelarm 23 besser seitlich geführt ist.

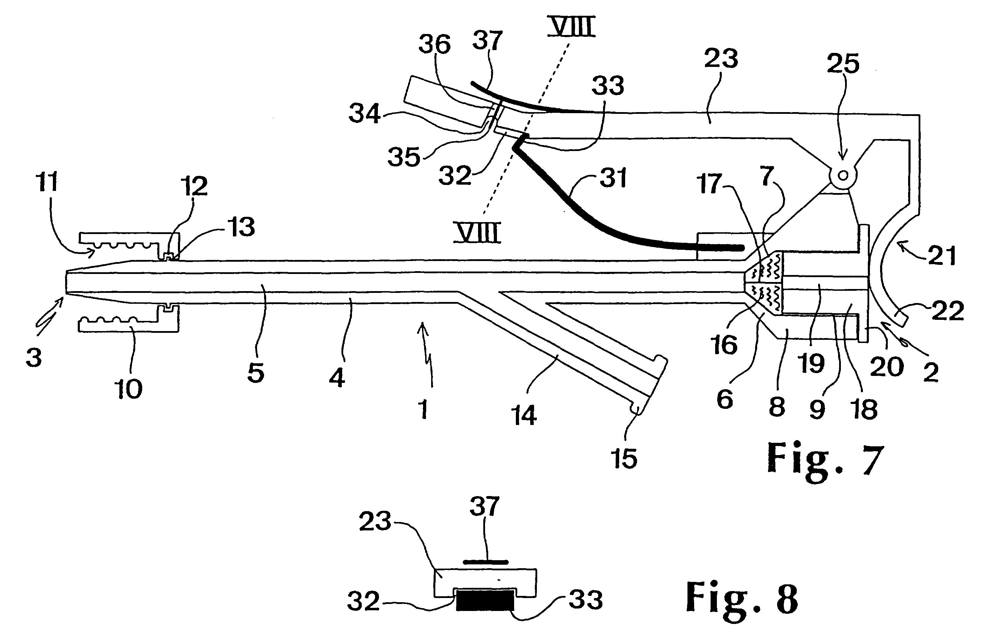

Fig. 7 zeigt ein weiteres Ausführungsbeispiel der Erfindung. Bei diesem Ausführungsbeispiel ist an dem dem Hebelarm 23 zugewandeten Ende der Blattfeder 31 ein vorstehender Steg 33 ausgebildet. Der Steg 33 gleitet in der Führungsnut 32, die zum Hebelende hin in eine im Hebelarm 23 eingebrachte Rastvertiefung 34 übergeht. Von der gegenüberliegenden Seite des Hebelarms 23 aus greift ein Stößel 35 durch eine Stößelausnehmung 36 in die Rastvertiefung 34 ein. Der Stößel 35 ist mittels einer Lösetaste 37 betätigbar.

Fig. 8 zeigt einen Querschnitt entlang der Linie VIII-VIII in Fig. 7.

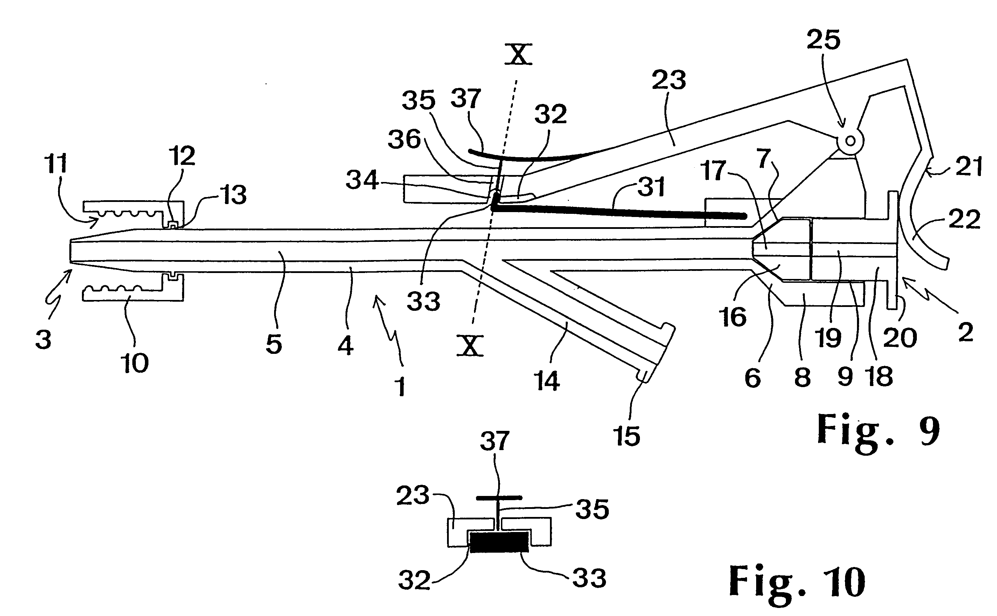

Fig. 9 zeigt das in Fig. 7 dargestellte Ausführungsbeispiel im geöffneten Zustand des Dichtventils. In diesem Zustand greift der Steg 33 in die Rastvertiefung 34 des gedrückten Hebelarms 23 ein. Der Stößel 35 ist aus der Rastvertiefung 34 gedrückt und spannt die Lösetaste 37 des gedrückten Hebelarms 23. Durch Betätigen der Lösetaste 37 läßt sich der Stößel 35 in die Rastvertiefung 34 drücken und damit die Arretierung des Hebelarmes 23 lösen.

Zweckmäßigerweise ist die Lösetaste 37 aus einer Blattfeder gefertigt, so daß sich die Lösetaste 37 im offenen Zustand des Dichtventils mit geringem Kraftaufwand betätigen läßt.

Bei einem abgewandelten Ausführungsbeispiel sind im Hebelarm 23 zwei oder mehrere Rastvertiefungen 34 ausgebildet, so daß sich das Dichtventil in zwei oder mehr Stufen öffnen oder schließen läßt.

Fig. 10 zeigt zur Veranschaulichung einen Querschnitt durch den Hebelarm 23 entlang der Linie X-X in Fig. 9.

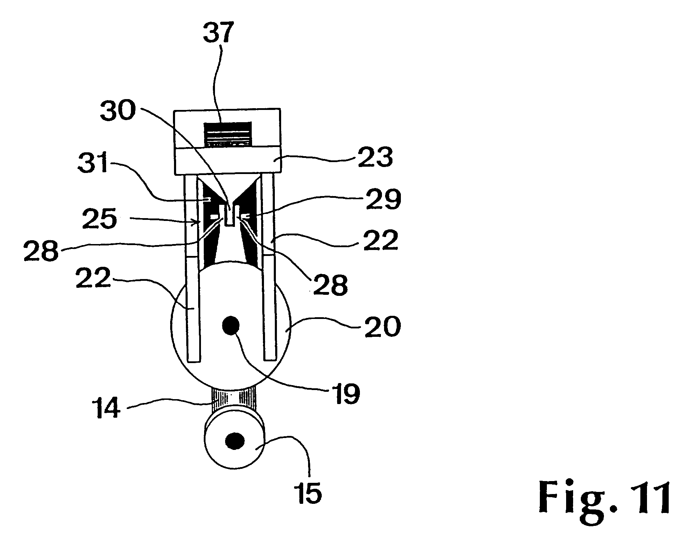

Fig. 11 zeigt zur Vervollständigung eine Ansicht der Dichtseite 2 des in den Fig. 7 bis 10 gezeigten Ausführungsbeispiels. Deutlich sind die Blattfeder 31 und die Lösetaste 37 zu sehen.

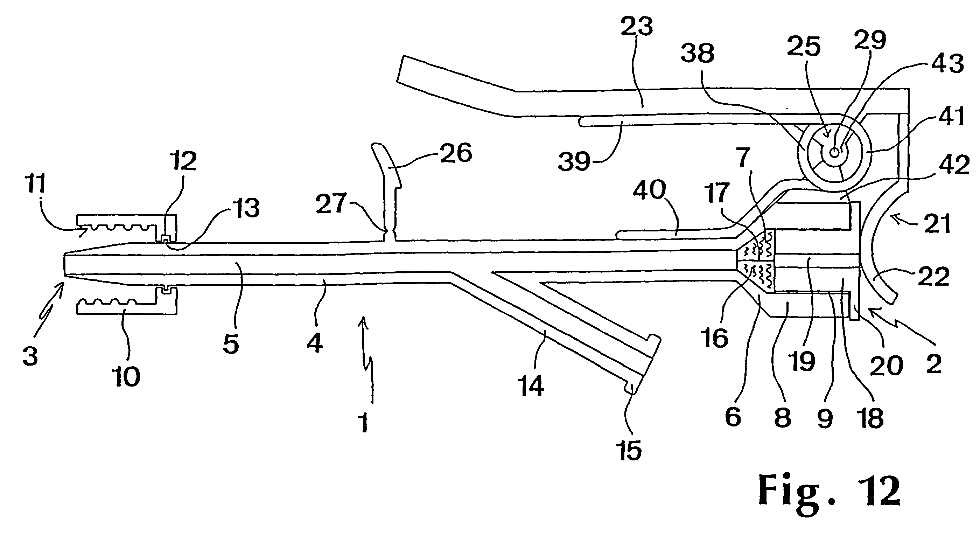

Fig. 12 schließlich stellt eine Querschnittsansicht eines weiteren Ausführungsbeispiels dar, bei dem eine Schenkelfeder 38 als Federelement dient. Diese Schenkelfeder 38 weist einen an Hebelarm 23 angebrachten Hebelarmschenkel 39 und einen am Ventilkörper 1 angebrachten Ventilkörperschenkel 40 sowie Windungen 41 auf, die den Gelenkstift 29 umschließen. Der Gelenkstift 29 ist von zwei seitlich der Schenkelfeder 38 am Dichtzylinder 8 angebrachte Gelenkstreben 42 gehalten. Zwei an Hebelarm 23 befestigte Gelenkösen 43 sind seitlich von der Schenkelfeder 38 auf dem Gelenkstift 29 verschwenkbar gelagert. Wie bei dem in den Fig. 1 bis 3 dargestellten Ausführungsbeispiel ist der Hebelarm 23 durch einen Schnapphaken 26 arretierbar.

Abschließend sei angemerkt, daß neben den Abwandlungen des Federmechanismus weitere Abwandlungen, insbesondere des Ventilkörpers 1, möglich sind. Beispielsweise ist es möglich, den Dichtzylinder 8 an das Hauptrohr 4 unmittelbar ohne die konische Erweiterung 6 anschließen zu lassen, so daß sich für einen Dichtpfropfen eine quer zur Längsachse verlaufende Anschlagsfläche ergibt. Diese Ausführungsform hat den Vorteil der größeren Einfachheit.

Eine weitere Möglichkeit ist, einen Dichtzylinder zu verwenden, der den gleichen Innendurchmesser wie das Hauptrohr 4 aufweist. Bei einem derartigen Ausführungsbeispiel kann ein Dichtpfropfen durch eine ringförmige Klebestelle im Dichtzylinder gehalten sein. Eine andere Möglichkeit ist, an einem Dichtpfropfen einen in Umfangsrichtung umlaufenden Steg vorzusehen, der in eine auf den Umfang der Innenseite des Dichtzylinders 8 umlaufende Nut eingreyft. Dabei darf jedoch die der abzudichtenden Flüssigkeit ausgesetzte Seite des Dichtpfropfens nicht zu groß gewählt werden, da ansonsten das Federelement eine zu große Gegendruckkraft aufbringen muß.

## Patentansprüche

1. Dichtventil mit einem langgestreckten, ein Anschlußende und ein Dichtende aufweisenden Ventilkörper (1), in dem eine sich vom Anschlußende (3) zum Dichtende (2) in axiale Richtung erstreckende Ventildurchführung (5, 9) ausgebildet ist, in der ein mit einer axialen Pfropfendurchführung (17) versehener, in axiale Richtung zum Dichten komprimierbarer Dichtpfropfen (16) angeordnet ist, **dadurch gekennzeichnet**, daß zum Komprimieren des Dichtpfropfens (16) ein durch einen Betätigungsmechanismus in seiner Wirkung neutralisierbares Federelement (24, 31, 38) vorgesehen ist.

2. Dichtventil nach Anspruch 1, dadurch gekennzeichnet, daß der Ventilkörper (1) einen Rohrkörper (4) aufweist, an den sich ein den Dichtpfropfen (16) umschließender Dichtzylinder (8) anschließt, dessen lichte Weite größer als die lichte Weite des Rohrkörpers (4) ist.

3. Dichtventil nach Anspruch 2, dadurch gekennzeichnet, daß in der Ventildurchführung (5, 9) im Übergangsbereich zwischen Rohrkörper (4) und Dichtzylinder (8) eine konische Anschlagsschulter (7) ausgebildet ist, an der der konisch angeschrägte Dichtpfropfen (16) anliegt.

4. Dichtventil nach einem der vorstehenden Ansprüche, dadurch gekennzeichnet, daß der Betätigungsmechanismus einen mit einer axialen Durchführung (19) versehenen, von der Dichtseite her in die Ventildurchführung (5, 9) eingreifenden Druckkolben (18) umfaßt, den der Betätigungsmechanismus gegen den Dichtpfropfen (16) drückt.

5. Dichtventil nach Anspruch 4, dadurch gekennzeichnet, daß der Betätigungsmechanismus einen längs zum Ventilkörper (1) verlaufenden, auf dem Ventilkörper (1) im Bereich des Dichtendes (2) verschwenkbar gelagerten Hebelarm (23) und eine an den Hebelarm (23) rechtwinklig angesetzte Druckgabel (21) aufweist, wobei das zwischen Ventilkörper (1) und Hebelarm (23) eingespannte Federelement (24, 31, 38) die Druckgabel (21) auf den Druckkolben (18) drückt.

6. Dichtventil nach Anspruch 5, dadurch gekennzeichnet, daß das Federelement eine Schraubendruckfeder (24) ist.

7. Dichtventil nach Anspruch 5, dadurch gekennzeichnet, daß das Federelement eine Schenkelfeder (38) ist.

8. Dichtventil nach Anspruch 5, dadurch gekennzeichnet, daß das Federelement eine Blattfeder (31) ist.

9. Dichtventil nach einem der Ansprüche 6 bis 8, dadurch gekennzeichnet, daß der Hebelarm (23) im geöffneten Zustand des Dichtventils in einen am Ventilkörper (1) angebrachten, wenigstens eine Raststufe aufweisenden Schnapphaken (26) einrastbar ist.

10. Dichtventil nach Anspruch 8, dadurch gekennzeichnet, daß die Blattfeder (31) am Hebelarm (23) in einer Führungsnut (32) geführt ist.

11. Dichtventil nach Anspruch 9 oder 10, dadurch gekennzeichnet, daß am Ende der Blattfeder (31) eine Erhöhung (33) ausgebildet ist, die im geöffneten Zustand des Dichtventils in wenigstens eine dazu passende Vertiefung (34) im Hebelarm (23) einrastbar ist.

12. Dichtventil nach Anspruch 11, dadurch gekennzeichnet, daß die Erhöhung (33) mit Hilfe eines mit einer Taste (37) betätigbaren Stößels (35) aus der Vertiefung (34) ausrastbar ist.

## Claims

1. A sealing valve comprising an elongate valve body (1) which has a connection end and a sealing end and in which there is provided a valve duct (5, 9) which extends in the axial direction from the connection end (3) to the sealing end (2) and in which there is arranged a sealing plug (16) which is provided with an axial plug duct (17) and which is compressible in the axial direction for sealing purposes, characterised in that a spring element (24, 31, 38) which can be neutralised in respect of its action by an actuating mechanism is provided for compression of the sealing plug (16).

2. A sealing valve according to claim 1 characterised in that the valve body (1) has a tubular body (4) adjoined by a sealing cylinder (8) which encloses the sealing plug (16) and the internal width of which is greater than the internal width of the tubular body (4).

3. A sealing valve according to claim 2 characterised in that formed in the valve duct (5, 9) in the transitional region between the tubular body (4) and the sealing cylinder (8) is a conical abutment shoulder (7) against which the conically bevelled sealing plug (16) bears.

4. A sealing valve according to one of the preceding claims characterised in that the actuating mechanism includes a pressure piston (18) which is provided with an axial duct (19) and which engages from the sealing side into the valve duct (5, 9) and which urges the actuating mechanism against the sealing plug (16).

5. A sealing valve according to claim 4 characterised in that the actuating mechanism has a lever arm (23) which extends lengthwise relative to the valve body (1) and which is mounted pivotably on the valve body (1) in the region of the sealing end (2), and a pressure fork (21) which is fitted at a right angle to the lever arm (23), wherein the spring element (24, 31, 38) which is stressed between the valve body (1) and the lever arm (23) presses the pressure fork (21) on to the pressure piston (18).

6. A sealing valve according to claim 5 characterised in that the spring element is a coil compression spring (24).

7. A sealing valve according to claim 5 characterised in that the spring element is a leg spring (38).

8. A sealing valve according to claim 5 characterised in that the spring element is a leaf spring (31).

9. A sealing valve according to one of claims 6 to 8 characterised in that in the opened condition of the sealing valve the lever arm (23) is engageable into a snap hook (26) which is mounted to the valve body (1) and which has at least one detent stage.

10. A sealing valve according to claim 8 characterised in that the leaf spring (31) is guided on the lever arm (23) in a guide groove (32).

11. A sealing valve according to claim 9 or claim 10 characterised in that provided at the end of the leaf spring (31) is a raised portion (33) which in the opened condition of the sealing valve is engageable into at least one recess (34) suitable for same in the lever arm (23).

12. A sealing valve according to claim 11 characterised in that the raised portion (33) is disengageable from the recess (34) by means of a pushrod (35) actuable by a press button (37).

## Revendications

1. Soupape d'étanchéité comprenant un corps de soupape (1) allongé présentant une extrémité de raccordement et une extrémité d'étanchéité, dans laquelle est conformé un guidage de soupape (5,9) s'étendant axialement de l'extrémité de branchement (3) à l'extrémité étanche (2), dans lequel est disposé un tampon d'étanchéité (16) présentant un guidage axial de tampon (17), compressible en direction axiale pour réaliser l'étanchéité, caractérisée en ce que pour comprimer le tampon d'étanchéité (16) est prévu un élément à force élastique (24,31,38) dont l'effet peut être neutralisé par un mécanisme d'actionnement.

2. Soupape d'étanchéité selon la revendication 1, caractérisée en ce que le corps de soupape (1) présente un corps tubulaire (4)prolongé par un cylindre d'étanchéité (8) entourant le tampon d'étanchéité (16), dont le diamètre intérieur est supérieur à celui du corps tubulaire (4).

3. Soupape d'étanchéité selon la revendication 2, caractérisée en ce qu'un épaulement conique de butée (7) est conformé dans le guidage de soupape (5,9) dans la zone de transition entre le corps tubulaire (4) et le cylindre d'étanchéité (8), épaulement avec lequel le tampon d'étanchéité (16) à rampe conique vient en contact.

4. Soupape d'étanchéité selon l'une quelconque des revendications précédentes, caractérisée en ce que le mécanisme d'actionnement comprend un piston de pression (18) muni d'un conduit axial (19) pénétrant dans le guidage de soupape (5,9) à partir du côté d'étanchéité, que le mécanisme d'actionnement presse contre le tampon d'étanchéité (16).

5. Soupape d'étanchéité selon la revendication 4, caractérisée en ce que le mécanisme d'actionnement présente un bras de levier (23) s'étendant longitudinalement par rapport au corps de soupape (1) et monté pivotant sur le corps de soupape (1) dans la zone de l'extrémité d'étanchéité (2), et un pied comprimeur (21) monté à angle droit sur le bras de levier (23), l'élément à force élastique (24,31,38) fixé entre le corps de soupape (1) et le bras de levier (23) pressant le pied comprimeur (21) sur le piston de pression (18).

6. Soupape d'étanchéité selon la revendication 5, caractérisée en ce que l'élément à force élastique est un ressort cylindrique de compression (24).

7. Soupape d'étanchéité selon la revendication 5, caractérisée en ce que l'élément à force élastique est un ressort à spirale (38).

8. Soupape d'étanchéité selon la revendication 5, caractérisée en ce que l'élément à force élastique est un ressort à lames (31).

9. Soupape d'étanchéité selon l'une quelconque des revendications 6 à 8, caractérisée en ce que lorsque la soupape d'étanchéité est à l'état ouvert, le bras de levier (23) peut être encliqueté dans une patte d'encliquetage (26) montée sur le corps de soupape (1) et présentant au moins un cran d'encliquetage.

10. Soupape d'étanchéité selon la revendication 8, caractérisée en ce que le ressort à lames (31) est guidé sur le bras de levier (23) dans une rainure de guidage (32).

11. Soupape d'étanchéité selon l'une quelconque des revendications 9 ou 10, caractérisée en ce qu'à l'extrémité du ressort à lames (31° est conformée une saillie (33) qui, dans la position ouverte de la soupape d'étanchéité, peut être encliquetée dans au moins un évidement (34) correspondant du bras de levier (23).

12. Soupape d'étanchéité selon la revendication 11, caractérisée en ce que la saillie (33) peut être désengagée de l'évidement (34) au moyen d'un coulisseau (35) actionné à l'aide d'une touche (37).
